# EUROPÄISCHE PATENTSCHRIFT

(11) **EP 2 794 017 B1**
(45) Veröffentlichungstag und Bekanntmachung des Hinweises auf die Patenterteilung: **19.09.2018**
(21) Anmeldenummer: 12790867.1
(22) Anmeldetag: 20.11.2012
(51) Int. Cl.: A61Q 5/06, A61K 8/35, A61K 8/37, A61K 8/92, A61K 8/41

(54) **MITTEL ZUM FÄRBEN VON KERATINISCHEN FASERN ENTHALTEND KATIONISCHE ANTHRACHINONFARBSTOFFE UND FETTSÄURETRIGLYCERIDE**
SUBSTANCE FOR DYEING KERATIN FIBERS, CONTAINING CATIONIC ANTHRAQUINONE DYES AND FATTY ACID TRIGLYCERIDES
AGENT DE COLORATION DE FIBRES KÉRATINIQUES CONTENANT DES COLORANTS ANTHRAQUINONIQUES CATIONIQUES ET DES TRIGLYCERIDES D'ACIDE GRAS

(30) Priorität: 20.12.2011 DE 102011089220
(43) Veröffentlichungstag der Anmeldung: 29.10.2014
(73) Patentinhaber: Henkel AG & Co. KGaA, 40589 Düsseldorf (DE)
(72) Erfinder: WESER, Gabriele, 41472 Neuss (DE); KOLONKO, Claudia, 42857 Remscheid (DE)
(86) Internationale Anmeldenummer: PCT/EP2012/073025
(87) Internationale Veröffentlichungsnummer: WO 2013/092060

(56) Entgegenhaltungen:
- EP-A1- 1 820 826
- EP-A1- 2 198 843
- WO-A1-2006/136303
- WO-A2-2008/022958
- CA-A1- 2 613 049

## Beschreibung

Die vorliegende Erfindung betrifft Mittel zum Färben und gegebenenfalls gleichzeitigen Aufhellen von keratinischen Fasern, insbesondere menschlichen Haaren, welche kationische Anthrachinonfarbstoffe und spezielle Fettsäuretriglyceride enthalten. Weiterhin betrifft die Erfindung die Verwendung dieser Mittel zur Erzeugung von Haarfärbungen mit erhöhtem Glanz, intensivem Farbergebnis, verbesserten Echtheitseigenschaften und verringerter Selektivität.

Für das Färben von keratinischen Fasern kommen im allgemeinen entweder direktziehende Farbstoffe oder Oxidationsfarbstoffe zur Anwendung. Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bei Personen mit empfindlicher Haut sensibilisierend wirken.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Direktziehende Farbstoffe werden unter schonenderen Bedingungen appliziert. Ihr Nachteil liegt jedoch darin, dass die Färbungen häufig nur über unzureichende Echtheitseigenschaften verfügen, insbesondere bei der Haarwäsche, aber auch gegenüber äußeren Einflüssen, wie Sonnenlicht oder reaktiven Umweltchemikalien, wie beispielsweise Schwimmbadwasser. Solche Färbungen sind auch gegen Shampoonieren in der Regel deutlich empfindlicher als die oxidativen Färbungen, so daß dann sehr viel schneller eine vielfach unerwünschte Nuancenverschiebung oder gar eine sichtbare Entfärbung eintritt.

Eine besondere Herausforderung für die Haarfärbung mit direktziehenden Farbstoffen stellt die gleichmäßige Färbung von häufig vorbehandeltem Haar, wie beispielsweise gebleichtem oder dauergewelltem Haar, dar, bei dem die Faser in den verschiedenen Längen oder verschieden behandelten Bereichen eine unterschiedlich starke Vorschädigung besitzt. Während der Färbung selbst kann das Färbemittel auf unterschiedlich vorgeschädigtem Haar ein ungleichmäßiges Färbeverhalten zeigen, aber auch durch wiederholte Haarwäschen können die Farbstoffe in den unterschiedlichen Haarbereichen verschieden stark auswaschen werden, wodurch ein uneinheitliches und damit unerwünschtes Farbergebnis erzielt wird.

Die Herstellung von Färbeformulierungen mit verringerter Selektivität, mittels welcher auf den unterschiedlich stark geschädigten Partien der Haare ein gleichmäßiges Farbergebnis erzielt werden kann, steht bei der Entwicklung Färbeprodukten auf Basis von Direktziehern daher nach wie vor besonders im Fokus. Insbesondere soll diese verringerte Selektivität nicht nur direkt nach dem Färbeprozeß, sondern auch nach wiederholten Haarwäschen noch vorhanden sein.

Die Aufgabe der vorliegenden Erfindung ist daher die Bereitstellung eines Färbemittels für keratinische Fasern, insbesondere menschliche Haare, welches neben anderen positiven Echtheitseigenschaften insbesondere eine geringe Selektivität (bzw. ein gutes Egalisiervermögen) und eine gute Waschechtheit besitzt.

Die mit den erfindungsgemäßen Mitteln erzeugten Färbungen sollen sowohl direkt nach dem Färbevorgang als auch nach wiederholten Haarwäschen ein brillantes und intensives Farbergebnis liefern. Hierbei sollen die Haare nach Anwendung des Färbemittels auch bei Vorhandensein von Haaren unterschiedlichen Schädigungsgrades gleichmäßig gefärbt sein, wobei diese Gleichmäßigkeit im Farbergebnis auch nach wiederholten Haarwäschen noch vorhanden sein soll.

Bei der vorliegenden Erfindung besteht die Zielsetzung vor allem auch darin, brillante und neutrale Nuancen im Blaubereich mit den oben beschriebenen vorteilhaften Echtheitseigenschaften bereit zu stellen, welche sich hervorragend für die Mattierung eignen. Zusätzlich soll der Glanz der Haare durch die Anwendung der Mittel erhöht werden und ein Austrocknen der Kopfhaut verhindert werden. Der Einsatz von kationischen Anthrachinonfarbstoffen in Produkten zum Färben von keratinischen Fasern ist prinzipiell bereits aus dem Stand der Technik, beispielsweise aus EP 1 006 154 B1 oder aus EP 1 820 826 A1, bekannt. Ferner werden in EP 2 329 809 Kombinationen von kationischen Anthrachinonfarbstoffen mit Oxidationsfarbstoffvorprodukten vom Entwicklertyp für die oxidative Färbung von Haaren beansprucht.

Die Kombinationen der kationischen Anthrachinone mit speziellen Fettsäuretriglyceriden sind im Stand der Technik bislang noch nicht beschrieben.

Im Zuge der zu dieser Erfindung führenden Arbeiten hat sich in überraschender Weise gezeigt, dass die Kombinationen von kationischen Anthrachinonfarbstoffen und speziellen Trifettsäureglyceriden zu Färbungen führen, welche die oben beschriebene Aufgabenstellung in herausragendem Maß erfüllen. Ein erster Gegenstand der vorliegenden Erfindung ist ein Mittel zum Färben und gegebenenfalls gleichzeitigen Aufhellen von keratinischen Fasern, insbesondere menschlichen Haaren, welches dadurch gekennzeichnet ist, dass es in einem kosmetischen Träger
(a) mindestens eine Verbindung der Formel (I) 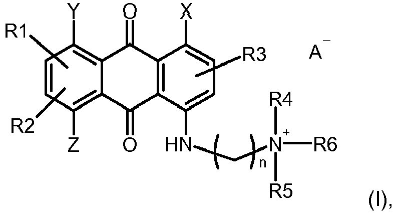 worin
   - R1, R2, R3: jeweils unabhängig voneinander für Wasserstoff, Halogen, eine Nitrogruppe, eine Cyanogruppe, eine Carboxylgruppe, eine Sulfonsäuregruppe, eine Hydroxygruppe, eine C₁-C₆-Acylaminogruppe, eine Carboxamidgruppe, eine Sulfonamidgruppe, eine C₁-C₆-Alkylgruppe, C₁-C₆-Alkoxygruppe, eine C₂-C₆-Alkenylgruppe, eine C₂-C₆-Hydroxyalkylgruppe oder eine C₁-C₆-Alkoxy-C₂-C₆-alkylgruppe stehen;
   - R4, R5, R6: jeweils unabhängig voneinander für eine C₁-C₆-Alkylgruppe, eine C₂-C₆-Alkenylgruppe, für eine C₂-C₆-Hydroxyalkylgruppe oder eine C₁-C₆-Alkoxy-C₂-C₆-alkylgruppe stehen;
   oder R4 und R5 bilden zusammen mit dem quartären Stickstoffatom, an das sie gebunden sind, einen 5-, 6- oder 7-gliedrigen Ring, der gegebenenfalls weitere Heteroatome enthalten kann und/oder weitere Substituenten tragen kann;
   - X, Y, Z: jeweils unabhängig voneinander für Wasserstoff, eine Hydroxygruppe oder eine Gruppe N(R7)(R8) stehen,
   worin
   R7 und R8 jeweils unabhängig voneinander für Wasserstoff, eine C₁-C₆-Alkylgruppe, eine C₂-C₆-Alkenylgruppe, für eine C₂-C₆-Hydroxyalkylgruppe oder eine C₁-C₆-Alkoxy- C₂-C₆-alkylgruppe stehen,
   unter der Maßgabe, dass mindestens einer der Reste X, Y, Z für eine Gruppe N(R7)(R8) steht,
   - n: für eine Zahl von 2 bis 6 steht,
   - A⁻: für ein physiologisch verträgliches Anion steht,
   und
(b) mindestens ein Fettsäuretriglycerid in einem Anteil von 0,1 bis 10,0 Gew.-%, bezogen auf das Gesamtgewicht des Mittels,
   enthält.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Der erfindungsgemäß verwendete Begriff "Färben von Keratinfasern" umfasst jedwede Form der Farbveränderung der Fasern. Umfasst sind insbesondere die unter den Begriffen Tönung, Aufhellung, Blondierung, Bleiche, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen.

Die erfindungsgemäßen Mittel enthalten das bzw. die kationischen Anthrachinone der Formel (I) und das bzw. die Fettsäuretriglyceride in einem kosmetischen Träger, bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind. Es ist aber auch denkbar, die erfindungsgemäßen Mittel in eine pulverförmige oder auch tablettenförmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Als ersten wesentlichen Inhaltsstoff (a) enthalten die erfindungsgemäßen Mittel mindestens einen direktziehenden kationischen Anthrachinonfarbstoff der allgemeinen Formel (I).

Die Substituenten R1 bis R8 der Verbindung der Formel (I) sind nachfolgend beispielhaft erläutert: Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine C₁-C₆-Hydroxyalkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Erfindungsgemäß bevorzugte C₁-C₆-Alkoxygruppen sind die Methoxy- oder die Ethoxygruppe. Beispiele für Halogenatome sind F-, Cl-, Br- oder I-Atome, wobei Br- oder Cl-Atome ganz besonders bevorzugt sind. Bevorzugte Beispiele für erfindungsgemäße C₁-C₆-Alkoxy-C₂-C₆-alkylgruppen sind die Methoxyethyl-, Ethoxyethyl-, Methoxypropyl-, Methoxybutyl-, Ethoxypropyl-, Ethoxybutyl- und die Methoxyhexylgruppe. Beispiele für eine C₁-C₆-Acylaminogruppe sind die Acetamidgruppe, die Propanamidgruppe und die Butanamidogruppe, wobei die Acetamidgruppe bevorzugt ist. Als bevorzugte Beispiele für einen 5-, 6- oder 7-gliedrigen Ring, der aus R4, R5 und dem quartären Stickstoffatom gebildet wird, sind der Pyrrolidiniumring, der Piperidiniumring, der Morpholiniumring und der 1-Azepaniumring zu nennen. Farbstoffe der Formel (I), in denen R1, R2 und R3 unabhängig voneinander für Wasserstoff, Halogen, eine Carboxylgruppe, eine Sulfonsäuregruppe, eine C₁-C₆-Alkylgruppe oder eine C₁-C₆-Alkoxygruppe stehen, liefern besonders intensive Färbeergebnisse und sind daher bevorzugt.

Weiterhin ist es bevorzugt, wenn einer der Reste ausgewählt aus R1, R2 und R3 für Halogen, eine Carboxylgruppe, eine Sulfonsäuregruppe, eine C₁-C₆-Alkylgruppe oder eine C₁-C₆-Alkoxygruppe steht und die anderen beiden Reste ausgewählt aus R1, R2 und R3 beide für Wasserstoff stehen. Eine bevorzugte Ausführungsform ist ein Mittel zum Färben und gegebenenfalls gleichzeitigen Aufhellen von keratinischen Fasern, welches dadurch gekennzeichnet ist, dass es eine Verbindung der Formel (I) enthält, worin wenigstens einer der Reste R1, R2 und/oder R3 für eine C₁-C₆-Alkylgruppe steht.

Bei besonders geeigneten Verbindungen der Formel (I) steht einer der Reste ausgewählt aus R1, R2 und R3 für eine C₁-C₆-Alkylgruppe und die anderen beiden Reste ausgewählt aus R1, R2 und R3 für Wasserstoff.

In einer explizit ganz besonders bevorzugten Ausführungsform stehen R1 und R2 beide für ein Wasserstoffatom, und R3 steht für eine Methylgruppe.

Des weiteren werden besonders mit den Mitteln gute Färbeergebnisse erzielt, die mindestens eine Verbindung der Formel (I) enthalten, bei welcher die Reste R4, R5 und R6 unabhängig voneinander für eine C₁-C₆-Alkylgruppe oder eine Alkenylgruppe stehen. Insbesondere bevorzugt steht jeder der Reste R4, R5 und R6 für eine C₁-C₆-Alkylgruppe.

Ganz besonders bevorzugt ist es, wenn R4 und R5 beide für eine Methylgruppe stehen und R6 für eine Methylgruppe, eine Ethylgruppe oder eine n-Propylgruppe steht.

Weiterhin ist es ganz besonders bevorzugt, wenn R4 und R5 beide für eine Methylgruppe stehen und R6 für eine n-Propylgruppe steht.

In einer ebenfalls besonders bevorzugten Ausführungsform steht jeder der Reste R4, R5 und R6 für eine Methylgruppe.

Bei Verbindungen der Formel (I) besteht die Maßgabe, dass mindestens einer der Reste X, Y, Z für eine Gruppe N(R7)(R8) steht. Es konnten insbesondere dann Färbungen mit guten anwendungstechnischen Eigenschaften erhalten werden, wenn Verbindungen der Formel (I) eingesetzt wurden, bei denen X für eine Gruppe N(R7)(R8) steht und Y und Z jeweils für Wasserstoff stehen.

R7 und R8 stehen bevorzugt unabhängig voneinander für Wasserstoff oder eine C₁-C₆-Alkylgruppe. Besonders bevorzugt stehen R7 und R8 unabhängig voneinander für Wasserstoff oder für eine Methylgruppe. Verbindungen der Formel (I), bei denen sowohl R7 als auch R8 für Wasserstoff stehen, haben sich als besonders geeignet erwiesen und sind daher besonders bevorzugt.

Im Rahmen der zu dieser Erfindung führenden Arbeiten hat es sich als ganz besonders vorteilhaft herausgestellt, wenn X für eine Gruppe NH₂ steht.

Eine weitere bevorzugte Ausführungsform ist daher ein Mittel zum Färben und gegebenenfalls gleichzeitigen Aufhellen von keratinischen Fasern, welches dadurch gekennzeichnet ist, dass es eine Verbindung der Formel (I) enthält, worin mindestens X für eine Gruppe NH₂ steht.

n steht bevorzugt für die Zahlen 2 oder 3 und ganz besonders bevorzugt für die Zahl 3.

A⁻ steht für ein physiologisch verträgliches Anion. Geeignete physiologisch verträgliche Anionen sind Halogenid, Hydrogensulfat, ½ Sulfat, Benzolsulfonat, p-Toluolsulfonat, Acetat, Citrat, Lactat, ½ Tartrat, Methylsulfat (H₃COSO₃⁻) oder Trifluormethansulfonat. Besonders bevorzugt steht A⁻ für Bromid oder für Methylsulfat (H₃COSO₃⁻), ganz besonders bevorzugt steht A⁻ für Methylsulfat (H₃COSO₃⁻).

Erfindungsgemäß bevorzugte Mittel zum Färben und gegebenenfalls gleichzeitigen Aufhellen keratinischer Fasern sind dadurch gekennzeichnet, dass sie mindestens eine Verbindung der allgemeinen Formel (I) enthalten, die ausgewählt ist aus
- 3-[(4-Amino-9,10-dihydro-3-methyl-9,10-dioxo-1-anthracenyl)amino]-N,N,N-trimethyl-1-propanaminiummethylsulfat,
- 3-[(4-Amino-9,10-dihydro-3-methyl-9,10-dioxo-1-anthracenyl)amino]-N,N,N-trimethyl-1-propanaminiumbromid,
- 3-[(4-Amino-9,10-dihydro-3-methyl-9,10-dioxo-1-anthracenyl)amino]-N,N,N-trimethyl-1-propanaminiumchlorid,
- 3-[(4-Amino-9,10-dihydro-3-methyl-9,10-dioxo-1-anthracenyl)amino]-N,N,N-trimethyl-1-propanaminium-p-toluolsulfonat,
- 3-[(4-Amino-9,10-dihydro-3-methyl-9,10-dioxo-1-anthracenyl)amino]-N,N,N-trimethyl-1-propanaminiumacetat,
- 3-{[9,10-Dihydro-4-(methylamino)-9,10-dioxo-1-anthracenyl]amino}-N,N-dimethyl-N-propyl-1-propanaminiummethylsulfat,
- 3-{[9,10-Dihydro-4-(methylamino)-9,10-dioxo-1-anthracenyl]amino}-N,N-dimethyl-N-propyl-1-propanaminiumbromid,
- 3-{[9,10-Dihydro-4-(methylamino)-9,10-dioxo-1-anthracenyl]amino}-N,N-dimethyl-N-propyl-1-propanaminiumchlorid,
- 3-{[9,10-Dihydro-4-(methylamino)-9,10-dioxo-1-anthracenyl]amino}-N,N-dimethyl-N-propyl-1-propanaminium-p-toluolsulfonat und
- 3-{[9,10-Dihydro-4-(methylamino)-9,10-dioxo-1-anthracenyl]amino}-N,N-dimethyl-N-propyl-1-propanaminiumacetat.

Als optimal geeignet zur Lösung der erfindungsgemäßen Aufgabenstellung hat sich die Verbindung der Formel (Ia) herausgestellt, 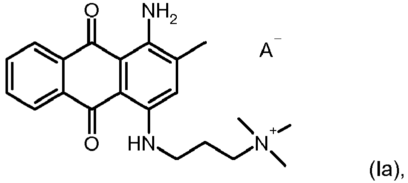 worin A⁻ für ein physiologisch verträgliches Anion, bevorzugt für Methylsulfat (H₃COSO₃⁻), steht. Eine weitere besonders bevorzugte Ausführungsform ist daher ein Mittel zum Färben und gegebenenfalls gleichzeitigen Aufhellen von keratinischen Fasern, welches dadurch gekennzeichnet ist, dass es als Verbindung der Formel (I) die Verbindung gemäß Formel (Ia) enthält, 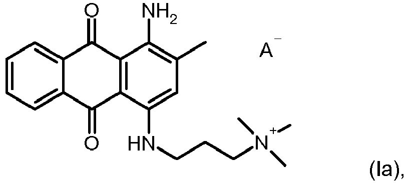 worin A⁻ für ein physiologisch verträgliches Anion, bevorzugt für Methylsulfat (H₃COSO₃⁻), steht.

Die erfindungsgemäßen Mittel zum Färben und gegebenenfalls gleichzeitigen Aufhellen von Keratinfasern enthalten die Verbindung(en) der Formel (I) vorzugsweise in Mengen oberhalb von 0,0001 Gew.-% und unterhalb von 5 Gew.-%, jeweils bezogen auf das gesamte Mittel.

Eine bevorzugte Ausführungsform ist dabei ein Mittel, welches die Verbindung(en) der Formel (I) in einem Anteil von 0,0001 bis 5 Gew.-%, bevorzugt 0,005 bis 3,5 Gew.-%, besonders bevorzugt 0,01 bis 2,5 Gew.-%, insbesondere 0,05 bis 1,5 Gew.-%, und insbesondere bevorzugt von 0,01 bis 1,0 Gew.-%, jeweils bezogen auf das Gesamtgewicht des Mittels, enthält.

Als zweiten wesentlichen Formulierungsbestandteil (b) enthalten die erfindungsgemäßen Mittel mindestens ein Fettsäuretriglycerid.

Unter einem Fettsäuretriglycerid wird im Sinne der vorliegenden Erfindung der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein.

Unter Fettsäuren sind erfindungsgemäß gesättigte oder ungesättigte, unverzweigte oder verzweigte, unsubstituierte oder substituierte C₈-C₂₄-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Besonders geeignete Vertreter von Fettsäuretriglyceriden (b) fallen in die allgemeine Formel (II), 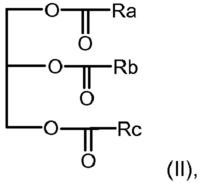 worin
- Ra, Rb, Rc: unabhängig voneinander für eine gesättigte oder ungesättigte, unverzweigte oder verzweigte, unsubstituierte oder substituierte C₇-C₂₃-Alkylgruppe stehen.

Es zeichnen sich die Fettsäuretriglyceride durch besondere Eignung aus, bei welchen mindestens eine der Estergruppen ausgehend von Glycerin mit einer gesättigten Fettsäure ausgebildet wird . Als gesättigte Fettsäure wird in diesem Zusammenhang bevorzugt eine Fettsäure ausgewählt aus

| | Fettsäure | Ra, Rb und/oder Rc = |
|---|---|---|
| F1 | Octansäure (Caprylsäure) | -C₇H₁₅ |
| F2 | Decansäure (Caprinsäure) | -C₉H₁₉ |
| F3 | Docdecansäure (Laurinsäure) | -C₁₁H₂₃ |
| F4 | Tetradecansäure (Myristinsäure) | -C₁₃H₂₇ |
| F5 | Hexadecansäure (Palmitinsäure) | -C₁₅H₃₁ |
| F6 | Octadecansäure (Stearinsäure) | -C₁₇H₃₅ |
| F7 | Eicosansäure (Arachinsäure) | -C₁₉H₃₉ |
| F8 | Docosansäure (Behensäure) | -C₂₁H₄₃ |
| F9 | Tetracosansäure (Lignocerinsäure) | -C₂₃H₄₇ |

eingesetzt.

Innerhalb dieser Ausführungsform ist es besonders vorteilhaft, wenn das Mittel ein Fettsäuretriglycerid (b) enthält, bei dem mindestens eine gesättigte C₁₄-C₂₀-Fettsäure einen Ester mit Glycerin ausbildet.

Damit sind die Verbindungen der allgemeinen Formel (II) besonders bevorzugt, bei denen mindestens einer der Reste ausgewählt aus Ra, Rb und/oder Rc für eine gesättigte, unverzweigte oder verzeigte, unsubstituierte oder substituierte C₁₃-C₁₉-Alkylgruppe steht.

Ganz besonders geeignet sind Verbindungen der Formel (II), bei denen mindestens einer der Reste ausgewählt aus Ra, Rb und/oder Rc für den Rest -C₁₃H₂₇(F4), -C₁₅H₃₁(F5), -C₁₇H₃₅ (F6) und/oder-C₁₉H₃₉ (F7) steht.

Des weiteren besitzen auch die Fettsäuretriglyceride eine herausragende Eignung, bei welchen mindestens eine der Estergruppen ausgehend von Glycerin mit einer ein- oder mehrfach ungesättigten Fettsäure ausgebildet wird. Als ungesättigte Fettsäure wird hierbei bevorzugt eine Fettsäure ausgewählt aus

| | Fettsäure | Ra, Rb und/oder Rc = |
|---|---|---|
| F11 | Petroselinsäure [(*Z*)-6-Octadecensäure]. | 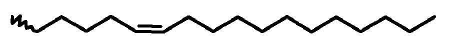 |
| F12 | Palmitoleinsäure [(9Z)-Hexadec-9-ensäure] | 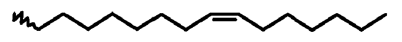 |
| F13 | Ölsäure [(9Z)-Octadec-9-ensäure] | 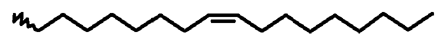 |
| F14 | Elaidinäsure [(9E)-Octadec-9-ensäure] | 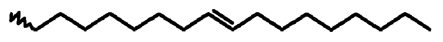 |
| F15 | Erucasäure [(13Z)-Docos-13-ensäure] | 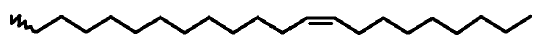 |
| F16 | Linolsäure [(9Z,12Z)-Octadeca-9,12-diensäure | 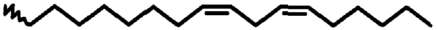 |
| F17 | Linolensäure [(9Z,12Z,15Z)-Octadeca-9,12,15-triensäure | 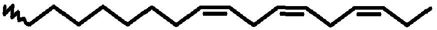 |
| F18 | Elaeostearinäure [(9Z,11E,13E)-Octadeca-9,11,3-triensäure] | 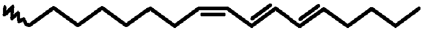 |
| F19 | Arachidonsäure [(5Z,8Z,11Z,14Z)-Icosa-5,8,11,14-tetraensäure] | 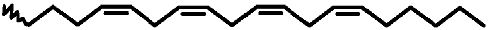 |
| F20 | Nervonsäure [(15Z)-Tetracos-15-ensäure] | 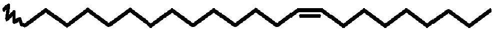 |

eingesetzt.

Im Zuge der zu dieser Erfindung führenden Arbeiten hat es sich weiterhin als bevorzugt herausgestellt, wenn das Färbemittel mindestens ein Fettsäuretriglycerid (b) enthält, bei dem mindestens eine Esterbindung des Glycerins mit einer ein-, zwei- oder dreifach ungesättigten C₁₆-C₂₀-Fettsäure ausgebildet wird. Insbesondere ist es bevorzugt, wenn mindestens eine der Fettsäuren ausgewählt aus Palmitoleinsäure (F12), Ölsäure (F13), Linolsäure (F16) und/oder Linolensäure (F17) mit Glycerin verestert sind.

Damit haben sich auch die Fettsäuretriglyceride der Formel (II) als besonders vorteilhaft herausgestellt, bei denen mindestens einer der Reste Ra, Rb und/oder Rc für einen der Reste F12, F13, F16 und/oder F17 steht.

Die zur Ausbildung der Ester in den Fettsäuretriglyceriden (b) eingesetzten Fettsäuren können auch einen oder mehrere Substituenten tragen. Hierbei tragen substituierte Fettsäuren bevorzugt einen oder mehrere Substituenten ausgewählt aus einer Hydroxygruppe, einer Carbonylgruppe und einer C₁-C₆-Alkoxygruppe. Bevorzugt sind die Fettsäuren mit einer Hydroxygruppe oder einer Methoxygruppe substituiert. Als substituierte Fettsäure wird bevorzugt eine Fettsäure ausgewählt aus

| | Fettsäure | Ra, Rb und/oder Rc = |
|---|---|---|
| F21 | Ricinolsäure [(12-Hydroxy-(Z)-octadec-9-ensäure | 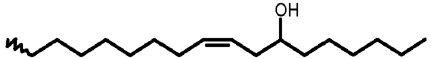 |
| F22 | 12-Hydroxy-octadecansäure | 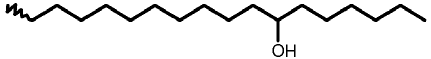 |

eingesetzt.

Fettsäuretriglyceride der Formel (II), bei denen mindestens einer der Reste Ra, Rb und/oder Rc für einen der Reste F21 oder F22 steht, sind besonders bevorzugt.

Eine weitere besonders bevorzugte Ausführungsform ist daher ein Mittel zum Färben und gegebenenfalls gleichzeitigen Aufhellen von keratinischen Fasern, welches dadurch gekennzeichnet ist, dass es als Fettsäuretriglycerid (b) mindestens eine Verbindung der allgemeinen Formel (II) enthält, 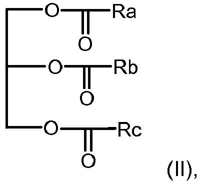 worin
- Ra, Rb, Rc: unabhängig voneinander für eine gesättigte oder ungesättigte, unverzweigte oder verzeigte, unsubstituierte oder substituierte C₇-C₂₃-Alkylgruppe stehen, wobei Ra, Rb und/oder Rc bevorzugt ausgewählt sind aus -C₁₃H₂₇(F4),-C₁₅H₃₁(F5), -C₁₇H₃₅ (F6), - C₁₉H₃₉ (F7) und den Resten F12, F13, F16, F17, F21 und F22.

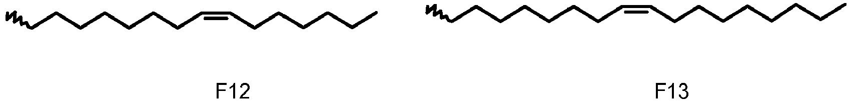

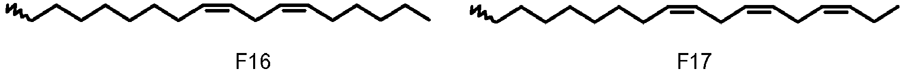

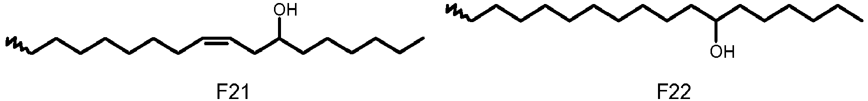

Eine weitere besonders bevorzugte Ausführungsform ist ein Mittel zum Färben und gegebenenfalls gleichzeitigen Aufhellen von keratinischen Fasern, welches dadurch gekennzeichnet ist, dass es als Fettsäuretriglycerid (b) mindestens eine Verbindung der allgemeinen Formel (II) enthält, in der mindestens einer der Reste Ra, Rb und/oder Rc für eine ein- oder mehrfach ungesättigte C₁₄-C₂₀-Alkylgruppe und/oder eine substituierte, bevorzugt mit einer Hydroxygruppe substituierte, C₁₄-C₂₀-Alkylgruppe steht.

In einer Ausführungsform der vorliegenden Erfindung enthält das Mittel als Fettsäuretriglycerid (b) mindestens einen Triester aus Glycerin und drei strukturgleichen Fettsäuren.

In einer weitern Ausführungsform der vorliegenden Erfindung enthält das erfindungsgemäße Mittel als Fettsäuretriglycerid (b) mindestens einen Triester aus Glycerin mit zwei unterschiedlichen Fettsäuren. Erfindungsgemäße Mittel, die ein Fettsäuretriglycerid (b) enthalten, welches durch Veresterung von drei unterschiedlichen Fettsäuren mit Glycerin erhalten wird, haben sich im Hinblick auf die anwendungtechnischen Eigenschaften des Färbemittels ebenfalls als vorteilhaft erwiesen. Bevorzugt werden die Triester aus Glycerin und den Fettsäuren ausgewählt, die mit den Verbindungen der Nr. 1 bis Nr. 863 in der Tabelle des Prioritätsdokumentes auf den Seiten 11 bis 36 offenbart sind.

Beispielsweise setzt sich die Struktur des Fettsäuretriglycerids Nr. 863 in der Tabelle des Prioritätsdokuments aus Glycerin und den Fettsäureresten Ra = F22, Rb = F21 und Rc = F17 zusammen, woraus Struktur (II₈₆₃) resultiert. Die Strukturen aller weiteren Derivate lassen sich analog ableiten.

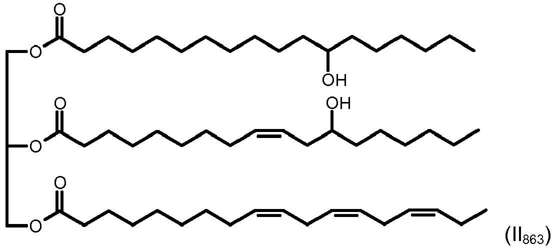

In einer weiteren ganz besonders bevorzugten Ausführungsform werden Gemische von verschiedenen Fettsäuretriglyceriden (b) eingesetzt. Es hat sich gezeigt, dass Öle und/oder Fette, deren Triglyceridfraktion eine spezielle Fettsäurenverteilung aufweist, die an diese Wirkstoffe gestellten Anforderungen in besonderem Maß erfüllen.

In diesem Zusammenhang besonders geeignete Gemische aus Fettsäuretriglyceriden (b) sind vor allem die Gemische, bei welchen die Zusammensetzungen aus Glycerin und verschiedenen Fettsäuren natürlich vorkommenden Ölen und Fetten entsprechen.

Die in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl vorkommenden Triglyceride bzw. deren Gemische sind zum Einsatz in den erfindungsgemäßen Mitteln besonders geeignet.

Sojaöl enthält ein Gemisch aus Triglyceriden, welches sich aus den Bestandteilen Palmitinsäure (F5), Stearinsäure (F6), Ölsäure (F13), Linolsäure (F16) und Linolensäure (F17) zusammensetzt. Da die Verwendung von Sojaöl in den erfindungsgemäßen Mitteln sich zur Erzeugung von intensiven und pflegenden Färbungen als besonders vorteilhaft erwiesen hat, sind Gemische aus Fettsäureglyceriden der Formel (II) besonders geeignet, bei denen die Reste Ra, Rb und/oder Rc für F5, F6, F13 und/oder F17 stehen.

Auch bei Einsatz von Erdnußöl in den erfindungsgemäßen Mitteln konnten brillante und intensive Färbungen mit besonderer Pflegewirkung und besonders guter Waschechtheit erhalten werden. Erdnußöl setzt sich hauptsächlich aus den Bestandteilen Palmitinsäure (F5), Stearinsäure (F6), Behensäure (F8), Lignocerinsäure (F9), Ölsäure (F13) und Linolsäure (F16) zusammen, welche mit Glycerin verestert vorliegen. Daher besitzen auch Mittel enthaltend ein Gemisch aus Fettsäureglyceriden der Formel (II) eine herausragende Eignung, bei denen die Reste Ra, Rb und/oder Rc für Fettsäurereste ausgewählt aus F5, F6, F8, F9, F13 und F16 stehen.

Bei Einsatz von Olivenöl in den erfindungsgemäßen Färbemitteln wurden ebenfalls Färbeergebnisse mit herausragender Waschechtheit und Pflegeleistung erhalten. Olivenöl enthält hauptsächlich aus den Bestandteilen Myristinsäure (F4), Palmitinsäure (F5), Ölsäure (F13) und Linolsäure (F16) aufgebaute Fettsäuretriglyceride. Färbemittel enthaltend ein Gemisch aus Fettsäuretriglyceriden der Formel (II), deren Reste Ra, Rb und/oder Rc aus F4, F5, F13 und/oder F16 ausgewählt sind, haben sich daher im Sinne der vorliegenden Erfindung ebenfalls als bevorzugt erwiesen.

Ebenfalls besondere Eignung zum Einsatz in den erfindungsgemäßen Färbemitteln zeigt Sonnenblumenöl. Sonnenblumenöl beinhaltet hauptsächlich Trifettsäureglyceride auf Basis von Palmitinsäure (F5), Stearinsäure (F6), Ölsäure (F13) und Linolsäure (F16). Damit sind Gemische von Fettsäuretriglyceridestern (b) der Formel (II) besonders bevorzugt, deren Reste Ra, Rb und/oder Rc ausgewählt sind aus F5, F6, F13 und F16.

Ferner eignet sich der Einsatz von Macadamianussöl in den erfindungsgemäßen Mitteln sehr gut, um farbstabile, besonders waschechte, pflegende und glänzende Färbungen auf keratinischen Fasern zu erzeugen. Bei den Trifettsäureglyceriden des Macadamianussöls bilden hauptsächlich die Fettsäuren Linolsäure (F16), Palmitinsäure (F5), Arachinsäure (F7), Stearinsäure (F6) die Esterbindungen mit Glycerin aus. Damit sind ebenfalls Gemische von Fettsäuretriglyceridestern (b) der Formel (II) besonders bevorzugt, bei denen Ra, Rb und/oder Rc ausgewählt sind aus den Fettsäureresten F16, F5, F7, und F6.

In Moringaöl ist ein Gemisch aus Fettsäuretriglyceriden enthalten, welches durch Veresterung von Glycerin mit den Fettsäuren Myristinsäure (F4), Palmitinsäure (F5), Stearinsäure (F6), Behensäure (F8), Ölsäure (F13) und Linolsäure (F16) erhalten wird. Damit sind ebenfalls die Gemische an Fettsäuretriglyceriden (b) der Formel (II) ganz besonders bevorzugt, bei denen Ra, Rb und/oder Rc ausgewählt sind aus den Fettsäureresten F4, F5, F6, F8, F13 und F16. Die mit Zusatz von Moringaöl erhaltenen Färbungen zeichnen sich ebenfalls durch glänzende Farbergebnisse und außerordentlich gute Waschechtheiten aus.

Aprikosenkernöl beinhaltet als Hauptbestandteile Triglyceride mit den Fettsäuren Ölsäure (F13) und Linolsäure (F16). Gemische aus Fettsäuretriglyceriden (b) der Formel (II), bei denen Ra, Rb und/oder Rc ausgewählt sind aus den Fettsäureresten F13 und F16 haben sich ebenfalls vorteilhaft auf Waschechtheit, Glanz und Griffgefühl der mit den erfindungsgemäßen Mitteln gefärbten Haare ausgewirkt.

Marulaöl beinhaltet im wesentlichen Fettsäuretriglyceride aus Glycerin und Palmitinsäure (F5), Stearinsäure (F6), Ölsäure (F13) und Linolsäure (F16) und zeigt ebenfalls außerordentlich gute Eignung zur Erzeugung von intensiven, glänzenden Färbungen mit exzellenter Waschechtheit. Gemische aus Fettsäuretriglyceriden (b) der Formel (II), bei denen Ra, Rb und/oder Rc ausgewählt sind aus den Fettsäureresten F5, F6, F13 und F16, sind aus diesem Grund ebenfalls bevorzugt. Schließlich haben sich auch Färbungen, die durch Einsatz der Kombinationen von kationischen Anthrachinonfarbstoffen der Formel (I) mit hydrogeniertem und/oder nicht hydrogeniertem Rizinusöl erhalten wurden, durch hervorragende Waschechtheiten, hohen Glanz und hohe Pflege ausgezeichnet.

Rizinusöl besteht zum größten Teil aus dem Triglycerid der Ricinolsäure (F21), welche durch Hydrogenierung in die 12-Hydroxy-octadecansäure (F22) überführt wird.

Damit sind weiterhin Gemische von Fettsäuretriglyceridestern (b) der Formel (II) ganz besonders bevorzugt, bei denen Ra, Rb und/oder Rc ausgewählt sind aus den Fettsäureresten von F21 und/oder F22.

Eine weitere besonders bevorzugte Ausführungsform ist daher ein Mittel zum Färben und gegebenenfalls gleichzeitigen Aufhellen von Haaren, welches dadurch gekennzeichnet ist, dass es als Fettsäuretriglycerid (b) ein natürlich vorkommendes Fettsäuretriglycerid und/oder Gemische natürlich vorkommender Fettsäuretriglyceride enthält, welche in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl enthalten sind.

Innerhalb der vorgenannten Gruppe sind das Macadamianussöl und das gegebenenfalls gehärtete Rizinusöl nochmals explizit als ganz besonders bevorzugt hervorzuheben.

Die erfindungsgemäßen Mittel zum Färben und/oder Aufhellen von keratinischen Fasern enthalten das bzw. die Fettsäuretriglyceride in einem Anteil von 0,1 bis 10,0 Gew.-%, insbesondere 0,5 bis 5,0 Gew.-%, und insbesondere bevorzugt von 0,75 bis 3,0 Gew.-%, jeweils bezogen auf das Gesamtgewicht des Mittels. Hierbei beziehen sich die angegebenen Gewichtsanteile auf die Gesamtmenge aller im Mittel enthaltenen Fettsäuretriglyceride.

Eine weitere bevorzugte Ausführungsform ist daher ein Mittel zum Färben und gegebenenfalls gleichzeitigen Aufhellen von keratinischen Fasern, welches dadurch gekennzeichnet ist, dass es das oder die Fettsäuretriglyceride in einem Anteil von 0,001 bis 15 Gew.-%, bevorzugt 0,05 bis 12 Gew.-%, besonders bevorzugt 0,1 bis 10,0 Gew.-%, insbesondere 0,5 bis 5,0 Gew.-%, und insbesondere bevorzugt von 0,75 bis 3,0 Gew.-%, jeweils bezogen auf das Gesamtgewicht des Mittels, enthält.

In einer weiteren bevorzugten Ausführungsform enthalten die erfindungsgemäßen Mittel neben dem Farbstoff der Formel (I) zusätzlich mindestens einen weiteren direktziehenden Farbstoff. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen. Die zusätzlichen direktziehenden Farbstoffe werden jeweils bevorzugt in einem Anteil von 0,001 bis 2 Gew.-%, bezogen auf die gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17 sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)-amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Färbeergebnisse mit herausragender Farbintensität, Brillanz und guter Waschechtheit werden insbesondere dann erhalten, wenn die erfindungsgemäßen Mittel als weiteren direktziehenden Farbstoff mindestens einen Farbstoff ausgewählt aus D&C Red No. 33 (Acid Red 33), Acid Black No. 1, D&C Orange No. 4 (Acid Orange No. 4), Acid Red 18, Basic Red 76, Acid Violet 43, HC Blue No. 12, N-(2-Hydroxethyl)-4-methyl-2-nitroanilin (Methyl Yellow), HC Yellow No. 2, Red B 54 und 2-Amino-6-chlor-4-phenol enthalten.

Die erfindungsgemäßen Mittel können weiterhin auch als Oxidationsfärbemittel eingesetzt werden. Solche Oxidationsfärbemittel enthalten zusätzlich mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Die direktziehenden Farbstoffe, Entwicklerkomponenten und Kupplerkomponenten werden bevorzugt jeweils in einer Menge von 0,0001 bis 5,0 Gew.-%, vorzugsweise 0,001 bis 2,5 Gew.-%, jeweils bezogen auf das anwendungsbereite Mittel, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Im Falle von Oxidationsfärbemitteln enthalten die Mittel bevorzugt ein Oxidationsmittel, bevorzugt Wasserstoffperoxid. Die Mengen an Wasserstoffperoxid entsprechen den Mengen in den erfindungsgemäßen Aufhellmitteln.

Die Färbemittel können weiterhin als aufhellende Färbemittel eingesetzt werden. Zur Erzielung des Aufhelleffekts enthalten die Mittel dazu Wasserstoffperoxid und/oder eines seiner festen Anlagerungsprodukte an organische oder anorganische Verbindungen.

Eine weitere Ausführungsform des ersten Erfindungsgegenstands ist daher dadurch gekennzeichnet, dass das Mittel zusätzlich Wasserstoffperoxid und/oder eines seiner festen Anlagerungsprodukte an organische oder anorganische Verbindungen enthält.

In einer bevorzugten Ausführungsform wird Wasserstoffperoxid selbst als wässrige Lösung verwendet. Vorzugsweise werden 6 bis 12 Gew.-%ige Lösungen in Wasser verwendet. Erfindungsgemäß bevorzugte anwendungsbereite Mittel des ersten Erfindungsgegenstands sind dadurch gekennzeichnet, dass sie, bezogen auf das Gesamtgewicht des anwendungsbereiten Mittels, 0,5 bis 20 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des Mittels, enthalten.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, in dem erfindungsgemäßen Mittel enthalten.

Eine weitere bevorzugte Ausführungsform ist ein Mittel, zum Färben und gleichzeitigen Aufhellen keratinischer Fasern, welches zusätzlich Wasserstoffperoxid, eines seiner festen Anlagerungsprodukte an organische oder anorganische Verbindungen, Ammoniumperoxodisulfat, Kaliumperoxodisulfat und/oder Natriumperoxodisulfat jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, enthält.

Die anwendungsbereiten Färbemittel können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die anwendungsbereiten Färbemittel als flüssige Zubereitung bereitgestellt und den Mitteln daher zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt.

Erfindungsgemäß bevorzugte Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugte Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß bevorzugte Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus der Amidoamine stellt das unter der Bezeichnung Tegoamid® S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Anteilen von 0,05 bis 10 Gew.-%, bezogen auf das gesamte Mittel, enthalten. Die anwendungsbereiten Färbemittel können weitere Hilfs- und Zusatzstoffe enthalten. So hat es sich als vorteilhaft erwiesen, wenn die Mittel mindestens ein Verdickungsmittel enthalten. bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind
- anionische Polymere,
- kationische, synthetische Polymere;
- natürlich vorkommende Verdickungsmittel, wie nichtionische Guargums, Skleroglucangums oder Gummi arabicum, Ghatti-Gummi, Karaya-Gummi, Tragant-Gummi, Carrageen-Gummi, Johannisbrotkernmehl, Pektine, Stärke-Fraktionen und Derivate wie Amylose, Amylopektin und Dextrine, sowie nichtionische Cellulosederivate, wie beispielsweise Methylcelluloseund Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Der pH-Wert der erfindungsgemäßen Mittel kann daher zwischen 3 und 11 liegen. Es ist bevorzugt, wenn der pH-Wert des anwendungsbereiten Mittels zwischen 5 und 11, insbesondere zwischen 5 und 7, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, Vitamine, Provitamine und Vitaminvorstufen; Pflanzenextrakte; Bienenwachs, Montanwachs und Paraffine; Quell- und Penetrationsstoffe wie Glycerin, Propylenglykolmonoethylether, Carbonate, Hydrogencarbonate, Guanidine, Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Zur Anwendung der erfindungsgemäßen Mittel eignet sich insbesondere ein Verfahren zum Färben von keratinischen Fasern, insbesondere menschlichen Haaren, welches dadurch gekennzeichnet ist, dass ein Mittel des ersten Erfindungsgegenstands auf die keratinhaltigen Fasern aufgebracht, 5 bis 60 Minuten auf der Faser belassen und anschließend mit Wasser wieder ausgespült oder mit einem Shampoo ausgewaschen wird. Bevorzugt beträgt die Einwirkzeit der anwendungsbereiten Färbemittel 5 bis 45 min, insbesondere 10 bis 40 min, besonders bevorzugt 15 bis 35 min. Nach Ende der Einwirkzeit wird die verbleibende Färbezubereitung mit Wasser oder einem Reinigungsmittel aus dem Haar gespült.

Die erfindungsgemäßen Mittel können als Einkomponentenmittel (Färbe- und Aufhellmittel) oder als Mehrkomponentenmittel wie Zweikomponentenmittel oder Dreikomponentenmittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Enthält das erfindungsgemäße Mittel sowohl direktziehende Farbstoffe - sowie gegebenenfalls zusätzlich Oxidationsfarbstoffvorprodukte - als auch Oxidationsmittel, so werden diese, um eine vorzeitige, unerwünschte Reaktion miteinander zu verhindern, zweckmäßigerweise getrennt voneinander konfektioniert und erst unmittelbar vor der Anwendung in Kontakt gebracht.

Ein Färbe- und Aufhellverfahren, bei dem die Färbecreme und das Oxidationsmittel zunächst getrennt vorliegen, ist daher bevorzugt. Ein weiterer Gegenstand der vorliegenden Erfindung ist daher ein Verfahren zum Färben und Aufhellen von menschlichen Haaren, bei dem eine Zusammensetzung auf wässriger Grundlage, enthaltend Wasserstoffperoxid, mit einem erfindungsgemäßen Mittel enthaltend mindestens eine Verbindung der Formel (I) zu einer homogenen Zusammensetzung vermischt, und diese auf das Haar aufgebracht wird. Das Fettsäuretriglycerid (b) kann in diesem Fall sowohl zusammen mit der Wasserstoffperoxid-Lösung als auch mit der Verbindung der Formel (I) konfektioniert werden.

In einer weiteren Ausführungsform der vorliegenden Erfindung sind daher Mittel bevorzugt, welche dadurch gekennzeichnet sind, dass sie unmittelbar vor der Anwendung durch Vermischen mindestens zweier Zubereitungen hergestellt werden, wobei die mindestens zwei Zubereitungen in mindestens zwei getrennt konfektionierten Containern bereitgestellt werden, und wobei ein Container ein Mittel (A), welches in einem kosmetischen Träger mindestens einen kationischen Anthrachinonfarbstoff der Formel (I) - sowie gegebenenfalls zusätzlich Oxidationsfarbstoffvorprodukte enthält, und ein weiterer Container eine Oxidationsmittelzubereitung (B), enthaltend mindestens ein Oxidationsmittel, enthält. Die Fettsäuretriglyceride (b) können hierbei sowohl zusammen mit dem kationischen Anthrachinonfarbstoff der Formel (I) in Container (A) als auch zusammen mit der Oxidationsmittelzubereitung in Container (B) konfektioniert werden.

Die Formulierung einer Kombination von (a) Verbindungen der allgemeinen Formel (I) mit (b) Fettsäuretriglyceriden eignet sich hervorragend zur Erzeugung von intensiven Färbungen mit hoher Brillanz, hohem Glanz und einer niedrigen Selektivität in Verbindung mit einer hervorragenden Waschechtheit. Die Mittel sind ebenfalls hervorragend geeignet, das Austrocknen der Kopfhaut zu minimieren oder zu verhindern.

Ein weiterer Gegenstand der vorliegenden Erfindung ist die Verwendung eines Mittels des ersten Erfindungsgegenstands zur Erzeugung von Haarfärbungen mit erhöhtem Glanz, intensivem Farbergebnis mit verbesserten Echtheitseigenschaften und/oder verringerter Selektivität.

Bezüglich weiterer bevorzugter Ausführungsformen der erfindungsgemäßen Verfahren und Verwendung gilt mutatis mutandis das zu den erfindungsgemäßen Mitteln Gesagte.

### Ausführunqsbeispiele

Es wurden die folgenden Rezepturen hergestellt. Die Mengenangaben verstehen sich, sofern nichts anderes vermerkt ist, jeweils in Gewichtsprozent.

### Formulierungsbeispiel 1

| **Beschreibung** | **Gew.-%** |
|---|---|
| Polyquaternium-10 | 0,45 |
| Citric acid Monohydrate | 0,70 |
| Timiron Super Silver | 0,20 |
| Cationic Blue 347 | 0,20 |
| Salicylic acid | 0,20 |
| Disodium Cocoamphodiacetate | 7,00 |
| Na-benzoate | 0,50 |
| Nicotinamide | 0,50 |
| Sodium Pyrrolidinon-2-carboxylate | 2,00 |
| Cutina HR | 1,00 |
| PEG-7 Glyceryl Cocoate | 0,50 |
| Sodium Myreth Sulfate (2 EO), 70% | 24,00 |
| NaOH, 50% | 0,15 |
| D-Panthenol, 75 % | 0,50 |
| Euperlan PK 3000 AM | 2,60 |
| ProSina | 2,00 |
| Sericin H | 0,20 |
| Caramel syrup, 75 % | 0,60 |
| Aprikosenkernöl | 0,10 |
| PEG-40 hydrogenated Castor Oil | 0,60 |
| Natriumchlorid | 0,20 |
| Antil 141 L | 1,00 |
| Hydrolyzed Silk Protein | 1,50 |
| Benzophenone-4 | 0,50 |
| Parfum | qs |
| Wasser | Ad 100 |

### Formulierungsbeispiel 2

| **Beschreibung** | **Gew.-%** |
|---|---|
| Cetearyl Alcohol | 6,00 |
| Coconut Alcohol | 6,00 |
| Ceteareth-12 | 2,00 |
| Ceteareth-20 | 3,00 |
| PEG-40 hydrogenated Castor Oil | 2,00 |
| Macadamianussöl | 1,00 |
| Methylparaben | 0,15 |
| Propylparaben | 0,15 |
| Tocopherol | 0,20 |
| Phenoxyethanol | 1,00 |
| HC Yellow No. 2 | 0,20 |
| N,N-Bis(2-hydroxyethyl)-2-nitro-p-phenylendiamin | 0,10 |
| Hydroxyethylcellulose | 1,00 |
| NaOH, 50% | 0,10 |
| Polyethylene Glycol MG 400 | 5,00 |
| HC Blue No. 2 | 1,50 |
| Basic Blue 99 | 0,42 |
| Basic Brown 16 | 0,08 |
| D&C Violet No.2 | 0,08 |
| Basic Red 76 | 0,08 |
| Basic Yellow 57 | 0,08 |
| D-Panthenol, 75 % | 0,30 |
| Citric acid Monohydrate | 0,10 |
| Cationic Blue 347 | 0,50 |
| Parfum | qs |
| Wasser | Ad 100 |

### Formulierungsbeispiel 3

| **Beschreibung** | **Gew.-%** |
|---|---|
| Cetearyl Alcohol | 6,00 |
| Coconut Alcohol | 6,00 |
| Ceteareth-12 | 2,00 |
| Ceteareth-20 | 3,00 |
| PEG-40 hydrogenated Castor Oil | 2,00 |
| Macadamianussöl | 1,00 |
| Methylparaben | 0,15 |
| Propylparaben | 0,15 |
| Tocopherol | 0,20 |
| Phenoxyethanol | 1,00 |
| Hydroxyethylcellulose | 1,00 |
| NaOH, 50 % | 0,10 |
| Polyethylene Glycol MG 400 | 5,00 |
| HC Blue No. 12 | 1,20 |
| Bluequat Bromide | 0,21 |
| Basic Brown 17 | 0,08 |
| D&C Violet 2 | 0,08 |
| Basic Red 76 | 0,08 |
| Basic Yellow 57 | 0,08 |
| HC Yellow No. 2 | 0,20 |
| N,N-Bis(2-hydroxyethyl)-2-nitro-p-phenylendiamin | 0,20 |
| D-Panthenol, 75 % | 0,30 |
| Citric acid Monohydrate | 0,10 |
| Cationic Blue 347 | 0,50 |
| Parfum | qs |
| Wasser | Ad 100 |

### Rezepturbestandteile

| | |
|---|---|
| Cationic Blue 347 | 3-[(4-Amino-9,10-dihydro-3-methyl-9,10-dioxo-1-anthracenyl)amino]-N,N,N-trimethyl-1-propanaminiummethylsulfat, |
| Bluequat Bromide | 3-{[9,10-Dihydro-4-(methylamino)-9,10-dioxo-1-anthracenyl]amino}-N,N-dimethyl-N-propyl-1-propanaminiumbromid |
| Timiron Super Silver | INCI-Bezeichnung: Mica, Titanium Dioxide (Merck KGaA) |
| Cutina HR | INCI-Bezeichnung: Hydrogenated Castor Oil (BASF) |
| Euperlan PK 3000 AM | ca. 43% Festsubstanz; INCI-Bezeichnung: Aqua, Glycol Distearate, Glycerin, Laureth-4, Cocamidopropyl Betaine, Formic Acid (BASF) |
| ProSina | INCI-Bezeichnung: Aqua, hydrolyzed Keratin (Keratec/Croda) |
| Sericin H | INCI-Bezeichnung: Sericin (Pentapharm) |
| Antil 141 L | ca. 40% Aktivsubstanz; INCI-Bezeichnung: Propylene Glycol, PEG-55 Propylene Glycol Oleate (Goldschmidt/Evonik) |

Die Färbeformulierungen wurden auf Haarsträhnen aufgetragen und dort für 30 Minuten bei Raumtemperatur belassen. Anschließend wurden die Fasern gründlich mit Wasser ausgespült und getrocknet.

Die behandelten Fasern zeichneten sich durch intensive Färbungen mit hohem Glanz, einer hervorragenden Waschechtheit und weichen Griff aus.

## Patentansprüche

1. Mittel zum Färben und gegebenenfalls gleichzeitigen Aufhellen von keratinischen Fasern, insbesondere menschlichen Haaren, enthaltend in einem kosmetischen Träger
(a) mindestens eine Verbindung der Formel (I) 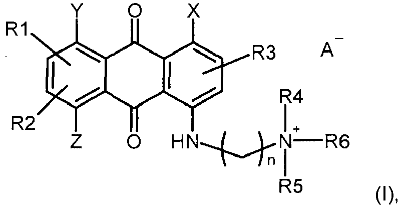 worin
R1, R2, R3 jeweils unabhängig voneinander für Wasserstoff, Halogen, eine Nitrogruppe, eine Cyanogruppe, eine Carboxylgruppe, eine Sulfonsäuregruppe, eine Hydroxygruppe, eine C₁-C₆-Acylaminogruppe, eine Carboxamidgruppe, eine Sulfonamidgruppe, eine C₁-C₆-Alkylgruppe, C₁-C₆-Alkoxygruppe, eine C₂-C₆-Alkenylgruppe, eine C₂-C₆-Hydroxyalkylgruppe oder eine C₁-C₆-Alkoxy-C₂-C₆-alkylgruppe stehen;
R4, R5, R6 jeweils unabhängig voneinander für eine C₁-C₆-Alkylgruppe, eine C₂-C₆-Alkenylgruppe, für eine C₂-C₆-Hydroxyalkylgruppe oder eine C₁-C₆-Alkoxy- C₂-C₆-alkylgruppe stehen;
oder R4 und R5 bilden zusammen mit dem quartären Stickstoffatom, an das sie gebunden sind, einen 5-, 6- oder 7-gliedrigen Ring, der gegebenenfalls weitere Heteroatome enthalten kann und/oder weitere Substituenten tragen kann;
X, Y, Z jeweils unabhängig voneinander für Wasserstoff, eine Hydroxygruppe oder eine Gruppe N(R7)(R8) stehen,
worin
R7 und R8 jeweils unabhängig voneinander für Wasserstoff, eine C₁-C₆-Alkylgruppe, eine C₂-C₆-Alkenylgruppe, für eine C₂-C₆-Hydroxyalkylgruppe oder eine C₁-C₆-Alkoxy- C₂-C₆-alkylgruppe stehen,
unter der Maßgabe, dass mindestens einer der Reste X, Y, Z für eine Gruppe N(R7)(R8) steht,
n für eine Zahl von 2 bis 6 steht,
A⁻ für ein physiologisch verträgliches Anion steht,
und
(b) mindestens ein Fettsäuretriglycerid,
wobei das Mittel das oder die Fettsäuretriqlvceride (b) in einem Anteil von 0,1 bis 10,0 Gew.-%, bezogen auf das Gesamtgewicht des Mittels, enthält.

2. Mittel nach Anspruch 1, **dadurch gekennzeichnet, dass** es eine Verbindung der Formel (I) enthält, worin wenigstens einer der Reste R1, R2 und/oder R3 für eine C₁-C₆-Alkylgruppe steht.

3. Mittel nach Anspruch 1 oder 2, **dadurch gekennzeichnet, dass** es eine Verbindung der Formel (I) enthält, worin mindestens X für eine Gruppe NH₂ steht.

4. Mittel nach einem der Ansprüche 1 bis 3, **dadurch gekennzeichnet, dass** es als Verbindung der Formel (I) die Verbindung gemäß Formel (Ia) enthält, 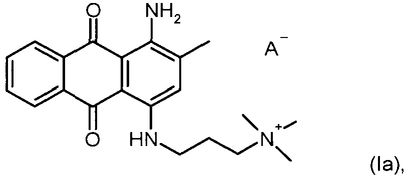 worin A⁻ für ein physiologisch verträgliches Anion, bevorzugt für Methylsulfat (H₃COSO₃⁻), steht.

5. Mittel nach einem der Ansprüche 1 bis 4, **dadurch gekennzeichnet, dass** es die Verbindung(en) gemäß Formel (I) in einem Anteil von 0,0001 bis 5 bevorzugt 0,005 bis 3,5 besonders bevorzugt 0,01 bis 2,5 Gew.-%, insbesondere 0,05 bis 1,5 und insbesondere bevorzugt von 0,01 bis 1,0 jeweils bezogen auf das Gesamtgewicht des Mittels, enthält.

6. Mittel nach einem der Ansprüche 1 bis 5, **dadurch gekennzeichnet, dass** es als Fettsäuretriglycerid (b) mindestens eine Verbindung der allgemeinen Formel (II) enthält, 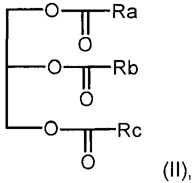 worin
Ra, Rb, Rc unabhängig voneinander für eine gesättigte oder ungesättigte, unverzweigte oder verzweigte, unsubstituierte oder substituierte C₇-C₂₃-Alkylgruppe stehen, wobei Ra, Rb und/oder Rc bevorzugt ausgewählt sind aus - C₁₃H₂₇, -C₁₅H₃₁,-C₁₇H₃₅, - C₁₉H₃₉ und den Resten F12, F13, F16, F17, F21 und F22

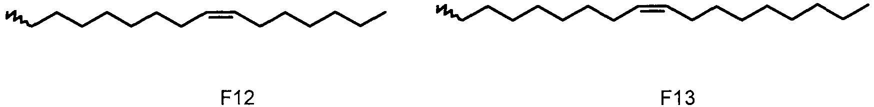

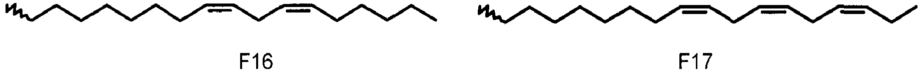

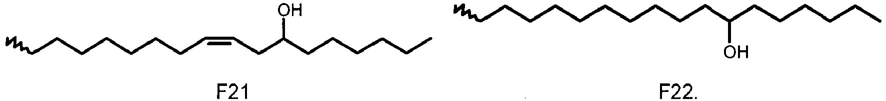

7. Mittel nach einem der Ansprüche 1 bis 6, **dadurch gekennzeichnet, dass** es als Fettsäuretriglycerid (b) mindestens eine Verbindung der allgemeinen Formel (II) enthält, in der mindestens einer der Reste Ra, Rb und/oder Rc für eine ein- oder mehrfach ungesättigte C₁₄-C₂₀-Alkylgruppe und/oder eine substituierte, bevorzugt mit einer Hydroxygruppe substituierte, C₁₄-C₂₀-Alkylgruppe steht.

8. Mittel nach einem der Ansprüche 1 bis 7, **dadurch gekennzeichnet, dass** es als Fettsäuretriglycerid(e) (b) ein natürlich vorkommendes Fettsäuretriglycerid und/oder Gemische natürlich vorkommender Fettsäuretriglyceride enthält, welche in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl enthalten sind.

9. Mittel nach einem der Ansprüche 1 bis 8, **dadurch gekennzeichnet ist, dass** es das oder die Fettsäuretriglyceride (b) in einem Anteil von 0,5 bis 5,0 Gew.-%, und insbesondere bevorzugt von 0,75 bis 3,0 jeweils bezogen auf das Gesamtgewicht des Mittels, enthält.

10. Mittel nach einem der Ansprüche 1 bis 9, **dadurch gekennzeichnet, dass** es neben einem Farbstoff der Formel (I) zusätzlich mindestens einen weiteren direktziehenden Farbstoff enthält.

11. Mittel nach Anspruch 10, **dadurch gekennzeichnet, dass** es als zusätzlichen direktziehenden Farbstoff mindestens einen kationischen Farbstoff aus der Gruppe Basic Blue 7, Basic Blue 26, Basic Violet 2, Basic Violet 14, Basic Yellow 57, Basic Red 76, Basic Blue 99, Basic Brown 16, Basic Brown 17, Basic Yellow 87, Basic Orange 31 und Basic Red 51 enthält.

12. Mittel nach einem der Ansprüche 10 oder 11, **dadurch gekennzeichnet, dass** es als zusätzlichen direktziehenden Farbstoff mindestens einen nichtionischen direktziehenden Farbstoff aus der Gruppe HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol enthält.

13. Mittel nach einem der Ansprüche 10 bis 12, **dadurch gekennzeichnet, dass** es als zusätzlichen direktziehenden Farbstoff mindestens einen Farbstoff aus der Gruppe D&C Red No. 33 (Acid Red 33), Acid Black No. 1, D&C Orange No. 4 (Acid Orange No. 4), Acid Red 18, Basic Red 76, Acid Violet 43, HC Blue No. 12, N-(2-Hydroxethyl)-4-methyl-2-nitroanilin (Methyl Yellow), HC Yellow No. 2, Red B 54 und 2-Amino-6-chlor-4-phenol enthält.

14. Mittel nach einem der Ansprüche 1 bis 13, **dadurch gekennzeichnet, dass** es einen pH-Wert zwischen 5 und 7 besitzt.

15. Verwendung eines Mittels nach einem der Ansprüche 1 bis 14 zur Erzeugung von Haarfärbungen mit verringerter Selektivität

## Claims

1. An agent for coloring and optionally simultaneously lightening keratin fibers, in particular human hair, containing, in a cosmetic carrier
(a) at least one compound of formula (I), a where 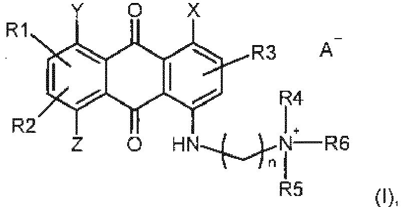
R1, R2, R3 each represent, independently of one another, hydrogen, halogen, a nitro group, a cyano group, a carboxyl group, a sulfonic acid group, a hydroxy group, a C₁-C₆ acyl amino group, a carboxamide group, a sulfonamide group, a C₁-C₆ alkyl group, C₁-C₆ alkoxy group, a C₂-C₆ alkenyl group, a C₂-C₆ hydroxyalkyl group or a C₁-C₆ alkoxy-C₂-C₆ alkyl group;
R4, R5, R6 each represent, independently of one another, a C₁-C₆ alkyl group, a C₂-C₆ alkenyl group, a C₂-C₆ hydroxyalkyl group or a C₁-C₆ alkoxy-C₂-C₆ alkyl group;
or R4 and R5, together with the quaternary nitrogen atom to which they are bonded, form a 5-, 6- or 7-membered ring, which can optionally contain additional heteroatoms and/or can carry additional substituents;
X, Y, Z each represent, independently of one another, hydrogen, a hydroxy group or
an N(R7)(R8) group,
where
R7 and R8 each represent, independently of one another, hydrogen, a C₁-C₆ alkyl group, a C₂-C₆ alkenyl group, a C₂-C₆ hydroxyalkyl group or a C₁-C₆ alkoxy-C₂-C₆ alkyl group,
with the proviso that at least one of the functional groups X, Y, Z represents an N(R7)(R8) group,
n represents a number from 2 to 6,
A⁻ represents a physiologically acceptable anion,
and
(b) at least one fatty acid triglyceride,
wherein the agent contains the fatty acid triglyceride(s) (b) in a proportion of from 0.1 to 10.0 wt.%, based on the total weight of the agent.

2. The agent according to claim 1, **characterized in that** it contains a compound of formula (I), where at least one of the functional groups R1, R2 and/or R3 represents a C₁-C₆ alkyl group.

3. The agent according to claim 1 or 2, **characterized in that** it contains a compound of formula (I), where at least X represents an NH₂ group.

4. The agent according to one of claims 1 to 3, **characterized in that** it contains the compound according to formula (la) as the compound of formula (I), 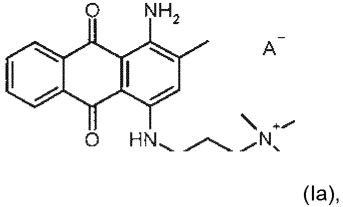 where A⁻ represents a physiologically acceptable anion, preferably methyl sulfate (H₃COSO₃⁻).

5. The agent according to one of claims 1 to 4, **characterized in that** it contains the compound(s) according to formula (I) in a proportion of from 0.0001 to 5 wt.%, preferably from 0.005 to 3.5 wt.%, particularly preferably from 0.01 to 2.5 wt.%, in particular from 0.05 to 1.5 wt.%, and particularly preferably from 0.01 to 1.0 wt.%, in each case based on the total weight of the agent.

6. The agent according to one of claims 1 to 5, **characterized in that** it contains at least one compound of general formula (II) as a fatty acid triglyceride (b), 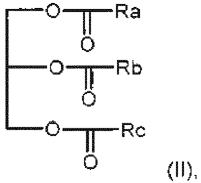 where
Ra, Rb, Rc represent, independently of one another, a saturated or unsaturated, unbranched or branched, unsubstituted or substituted C₇-C₂₃ alkyl group, Ra, Rb and/or Rc preferably being selected from -C₁₃H₂₇, -C₁₅H₃₁, -C₁₇H₃₅, -C₁₉H₃₉ and the functional groups F12, F13, F16, F17, F21 and F22.

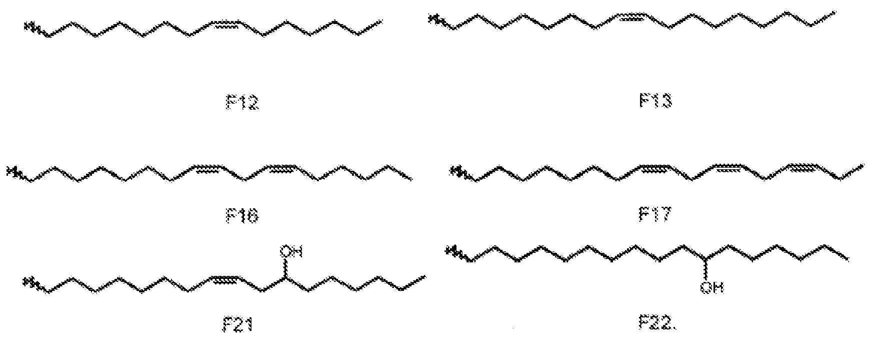

7. The agent according to one of claims 1 to 6, **characterized in that** it contains at least one compound of general formula (II) as a fatty acid triglyceride (b), in which compound at least one of the functional groups Ra, Rb and/or Rc represents a mono- or polyunsaturated C₁₄-C₂₀ alkyl group and/or a substituted C₁₄-C₂₀ alkyl group, preferably substituted with a hydroxy group.

8. The agent according to one of claims 1 to 7, **characterized in that** it contains a naturally occurring fatty acid triglyceride and/or mixtures of naturally occurring fatty acid triglycerides as the fatty acid triglyceride(s) (b), which naturally occurring fatty acid triglycerides are contained in soybean oil, groundnut oil, olive oil, sunflower oil, macadamia nut oil, moringa oil, apricot kernel oil, marula oil and/or optionally hydrogenated castor oil.

9. The agent according to one of claims 1 to 8, **characterized in that** it contains the fatty acid triglyceride(s) (b) in a proportion of from 0.5 to 5.0 wt.%, and particularly preferably from 0.75 to 3.0 wt.%, in each case based on the total weight of the agent.

10. The agent according to one of claims 1 to 9, **characterized in that** it additionally contains, in addition to a dye of formula (I), at least one further direct dye.

11. The agent according to claim 10, **characterized in that** it contains, as the additional direct dye, at least one cationic dye from the group comprising Basic Blue 7, Basic Blue 26, Basic Violet 2, Basic Violet 14, Basic Yellow 57, Basic Red 76, Basic Blue 99, Basic Brown 16, Basic Brown 17, Basic Yellow 87, Basic Orange 31 and Basic Red 51.

12. The agent according to one of claims 10 or 11, **characterized in that** it contains, as the additional direct dye, at least one non-ionic direct dye from the group comprising HC Yellow 2, HC Yellow 4, HC Yellow 5, HC Yellow 6, HC Yellow 12, HC Orange 1, Disperse Orange 3, HC Red 1, HC Red 3, HC Red 7, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-diamino-2-nitrobenzene, 2-amino-4-nitrophenol, 1,4-bis-(2-hydroxyethyl)amino-2-nitrobenzene, 3-nitro-4-(2-hydroxyethyl)aminophenol, 2-(2-hydroxyethyl)amino-4,6-dinitrophenol, 4-[(2-hydroxyethyl)amino]-3-nitro-1-methylbenzene, 1-amino-4-(2-hydroxyethyl)amino-5-chloro-2-nitrobenzene, 4-amino-3-nitrophenol, 1-(2'-ureidoethyl)amino-4-nitrobenzene, 2-[(4-amino-2-nitrophenyl)amino]benzoic acid, 4-[(3-hydroxypropyl)amino]-3-nitrophenol, 4-nitro-o-phenylenediamine, 6-nitro-1,2,3,4-tetrahydroquinoxaline, 2-hydroxy-1,4-naphthoquinone, picramic acid and the salts thereof, 2-amino-6-chloro-4-nitrophenol, 4-ethylamino-3-nitrobenzoic acid and 2-chloro-6-ethylamino-4-nitrophenol.

13. The agent according to one of claims 10 to 12, **characterized in that** it contains, as the additional direct dye, at least one dye from the group comprising D&C Red No. 33 (Acid Red 33), Acid Black No. 1, D&C Orange No. 4 (Acid Orange No. 4), Acid Red 18, Basic Red 76, Acid Violet 43, HC Blue No. 12, N-(2-hydroxyethyl)-4-methyl-2-nitroaniline (Methyl Yellow), HC Yellow No. 2, Red B 54 and 2-amino-6-chloro-4-phenol.

14. The agent according to one of claims 1 to 13, **characterized in that** it has a pH of between 5 and 7.

15. The use of an agent according to one of claims 1 to 14 for producing hair dyes having reduced selectivity.

## Revendications

1. Agent de coloration et éventuellement d'éclaircissement simultané de fibres kératiniques, en particulier de cheveux humains, contenant, dans un véhicule cosmétique,
(a) au moins un composé de formule (I) 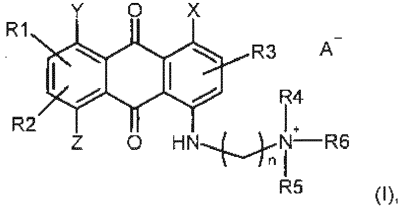 dans laquelle
R1, R2, R3 représentent chacun indépendamment les uns des autres l'hydrogène, un halogène, un groupe nitro, un groupe cyano, un groupe carboxyle, un groupe acide sulfonique, un groupe hydroxy, un groupe acylamino en C₁-C₆, un groupe carboxamide, un groupe sulfonamide, un groupe alkyle en C₁-C₆, un groupe alcoxy en C₁-C₆, un groupe alcényle en C₂-C₆, un groupe hydroxyalkyle en C₂-C₆ ou un groupe alcoxy en C₁-C₆-alkyle en C₂-C₆ ;
R4, R5, R6 représentent chacun indépendamment les uns des autres un groupe alkyle en C₁-C₆, un groupe alcényle en C₂-C₆, un groupe hydroxyalkyle en C₂-C₆ ou un groupe alcoxy en C₁-C₆-alkyle en C₂-C₆ ;
ou R4 et R5 forment ensemble avec l'atome d'azote quaternaire auquel ils sont liés, un cycle à 5, 6 ou 7 chaînons qui peut éventuellement contenir d'autres hétéroatomes et/ou peut porter d'autres substituants ;
X, Y, Z représentent chacun indépendamment les uns des autres l'hydrogène, un groupe hydroxy ou un groupe N(R7)(R8),
dans lequel
R7 et R8 représentent chacun indépendamment l'un de l'autre l'hydrogène, un groupe alkyle en C₁-C₆, un groupe alcényle en C₂-C₆, un groupe hydroxyalkyle en C₂-C₆ ou un groupe alcoxy en C₁-C₆-alkyle en C2-C6,
à condition qu'au moins un des radicaux X, Y, Z représente un groupe N(R7)(R8),
n représente un nombre allant de 2 à 6,
A⁻ représente un anion physiologiquement compatible,
et
(b) au moins un triglycéride d'acide gras,
l'agent contenant le ou les triglycérides d'acide gras (b) dans une proportion de 0,1 à 10,0% en poids, par rapport au poids total de l'agent.

2. Agent selon la revendication 1, **caractérisé en ce qu'**il contient un composé de formule (I), dans laquelle au moins un des radicaux R1, R2 et/ou R3 représente un groupe alkyle en C₁-C₆.

3. Agent selon la revendication 1 ou 2, **caractérisé en ce qu'**il contient un composé de formule (I), dans laquelle au moins X représente un groupe NH₂.

4. Agent selon l'une des revendications 1 à 3, **caractérisé en ce qu'**il contient, comme composé de formule (I), le composé selon la formule (Ia), 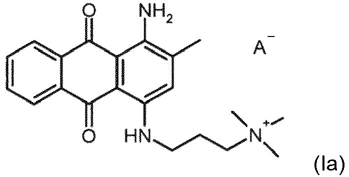 dans laquelle A⁻ représente un anion physiologiquement compatible, de préférence le méthylsulfate (H₃COSO₃⁻).

5. Agent selon l'une des revendications 1 à 4, **caractérisé en ce qu'**il contient le ou les composé(s) selon la formule (I) dans une proportion de 0,0001 à 5% en poids, de préférence de 0,005 à 3,5% en poids, de manière davantage préférée de 0,01 à 2,5% en poids, en particulier de 0,05 à 1,5% en poids, et de manière particulièrement préférée de 0,01 à 1,0% en poids, par rapport au poids total de l'agent respectivement.

6. Agent selon l'une des revendications 1 à 5, **caractérisé en ce qu'**il contient comme triglycéride d'acide gras (b) au moins un composé de formule générale (II), 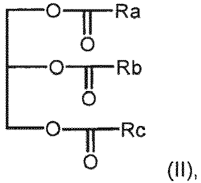 dans laquelle
Ra, Rb, Rc représentent, de manière indépendante, un groupe alkyle en C₇-C₂₃ saturé ou insaturé, non ramifié ou ramifié, non substitué ou substitué, Ra, Rb et/ou Rc étant de préférence choisis parmi - C₁₃H₂₇, -C₁₅H₃₁, -C₁₇H₃₅,-C₁₉H₃₉ et les radicaux F12, F13, F16, F17, F21 et F22

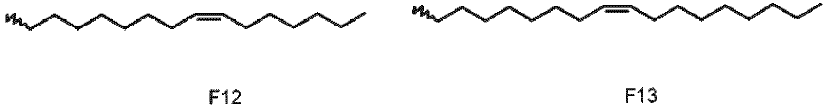

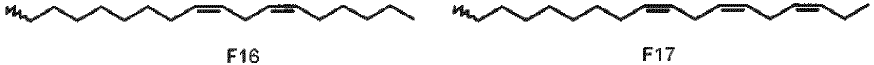

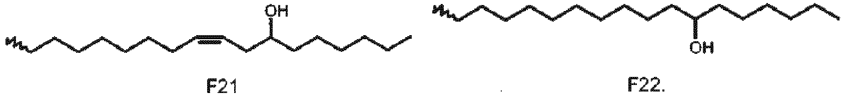

7. Agent selon l'une des revendications 1 à 6, **caractérisé en ce qu'**il contient, comme triglycéride d'acide gras (b), au moins un composé de formule générale (II), dans laquelle au moins un des radicaux Ra, Rb et/ou Rc représente un groupe alkyle en C₁₄-C₂₀ mono ou polyinsaturé et/ou un groupe alkyle en C₁₄-C₂₀ substitué, de préférence substitué par un groupe hydroxy.

8. Agent selon l'une des revendications 1 à 7, **caractérisé en ce qu'**il contient comme triglycéride(s) d'acide gras (b) un triglycéride d'acide gras d'origine naturelle et/ou des mélanges de triglycérides d'acide gras d'origine naturelle, présents dans l'huile de soja, l'huile d'arachide, l'huile d'olive, l'huile de tournesol, l'huile de noix de macadamia, l'huile de moringa, l'huile de noyau d'abricot, l'huile de marula et/ou éventuellement l'huile de ricin durcie.

9. Agent selon l'une des revendications 1 à 8, **caractérisé en ce qu'**il contient le ou les triglycéride(s) d'acide gras (b) dans une proportion de 0,5 à 5,0% en poids, et de manière particulièrement préférée de 0,75 à 3,0% en poids, par rapport au poids total de l'agent respectivement.

10. Agent selon l'une des revendications 1 à 9, **caractérisé en ce qu'**il contient en plus d'un colorant de formule (I) au moins un autre colorant à action directe.

11. Agent selon la revendication 10, **caractérisé en ce qu'**il contient, comme colorant à action directe supplémentaire, au moins un colorant cationique issu de l'ensemble comprenant Basic Blue 7, Basic Blue 26, Basic Violet 2, Basic Violet 14, Basic Yellow 57, Basic Red 76, Basic Blue 99, Basic Brown 16, Basic Brown 17, Basic Yellow 87, Basic Orange 31 et Basic Red 51.

12. Agent selon l'une des revendications 10 ou 11, **caractérisé en ce qu'**il contient, comme colorant à action directe supplémentaire, au moins un colorant à action directe non ionique issu de l'ensemble comprenant HC Yellow 2, HC Yellow 4, HC Yellow 5, HC Yellow 6, HC Yellow 12, HC Orange 1, Disperse Orange 3, HC Red 1, HC Red 3, HC Red 7, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, le 1,4-diamino-2-nitrobenzène, le 2-amino-4-nitrophénol, le 1,4-bis-(2-hydroxyéthyl)-amino-2-nitrobenzène, le 3-nitro-4-(2-hydroxyéthyl)-aminophénol, le 2-(2-hydroxyéthyl)amino-4,6-dinitrophénol, le 4-[(2-hydroxyéthyl)amino]-3-nitro-1-méthylbenzène, le 1-amino-4-(2-hydroxyéthyl)-amino-5-chloro-2-nitrobenzène, le 4-amino-3-nitrophénol, le 1-(2'-uréidoéthyl)amino-4-nitrobenzène, l'acide 2-[(4-amino-2-nitrophényl)amino]-benzoïque, le 4-[(3-hydroxypropyl)amino]-3-nitrophénol, la 4-nitro-o-phénylènediamine, la 6-nitro-1,2,3,4-tétrahydroquinoxaline, la 2-hydroxy-1,4-naphtoquinone, l'acide picramique et ses sels, le 2-amino-6-chloro-4-nitrophénol, l'acide 4-éthylamino-3-nitrobenzoïque et le 2-chloro-6-éthylamino-4-nitrophénol.

13. Agent selon l'une des revendications 10 à 12, **caractérisé en ce qu'**il contient, comme colorant à action directe supplémentaire, au moins un colorant issu de l'ensemble comprenant D&C Red No. 33 (Acid Red 33), Acid Black No. 1, D&C Orange No. 4 (Acid Orange No. 4), Acid Red 18, Basic Red 76, Acid Violet 43, HC Blue No. 12, la N-(2-hydroxéthyl)-4-méthyl-2-nitroaniline (jaune de méthyle), HC Yellow No. 2, Red B 54 et le 2-amino-6-chloro-4-phénol.

14. Agent selon l'une des revendications 1 à 13, **caractérisé en ce qu'**il possède un pH compris entre 5 et 7.

15. Utilisation d'un agent selon l'une des revendications 1 à 14 pour la fabrication de colorations pour cheveux ayant une sélectivité réduite.
